# EUROPEAN PATENT SPECIFICATION

(11) **EP 2 413 848 B1**
(45) Date of publication and mention of the grant of the patent: **20.03.2013**
(21) Application number: 10722859.5
(22) Date of filing: 31.03.2010
(51) Int. Cl.: A61F 2/92, B29C 53/40, B29C 55/02, B29C 35/16

(54) **Method of manufacturing stents**
Verfahren zur Herstellung von Stents
Procédé de fabrication de stents

(30) Priority: 31.03.2009 US 415841
(43) Date of publication of application: 08.02.2012
(73) Proprietor: Abbott Cardiovascular Systems Inc., Santa Clara, CA 95054-2807 (US)
(72) Inventor: WANG, Yunbing, Sunnyvale California 94089 (US)
(74) Representative: ZBM Patents
(86) International application number: PCT/US2010/029442
(87) International publication number: WO 2010/120532

(56) References cited:
- WO-A1-2007/149081
- US-A1- 2007 207 186

## Description

### BACKGROUND OF THE INVENTION

### Field of the Invention

This invention relates to a method of manufacturing polymeric medical devices, in particular, stents.

### Description of the State of the Art

This invention relates to radially expandable endoprostheses, that are adapted to be implanted in a bodily lumen. An "endoprosthesis" corresponds to an artificial device that is placed inside the body. A "lumen" refers to a cavity of a tubular organ such as a blood vessel. A stent is an example of such an endoprosthesis. Stents are generally cylindrically shaped devices that function to hold open and sometimes expand a segment of a blood vessel or other anatomical lumen such as urinary tracts and bile ducts. Stents are often used in the treatment of atherosclerotic stenosis in blood vessels. "Stenosis" refers to a narrowing or constriction of a bodily passage or orifice. In such treatments, stents reinforce body vessels and prevent restenosis following angioplasty in the vascular system. "Restenosis" refers to the reoccurrence of stenosis in a blood vessel or heart valve after it has been treated (as by balloon angioplasty, stenting, or valvuloplasty) with apparent success.

Several different types of stent designs have been devised to perform the treatment described above. One class of designs includes stents that are composed of scaffolding that includes a pattern or network of interconnecting structural elements or struts, formed from wires, tube stock, or sheets of material rolled into a cylindrical shape. This scaffolding gets its name because it physically holds open and, if desired, expands the wall of the passageway. The stent scaffolding is compressed or crimped onto a catheter to a diameter so that they can be delivered to and expanded or deployed at a treatment site. The compression and expansion of the scaffolding in such designs is achieved by flexing or bending of structural elements. The crimping and expansion of the scaffolding generally involves plastic deformation of the flexed or bent elements, which facilitates retention or locking of the stent in the crimped or expanded configuration.

In another type of design the stent is made of a rolled up sheet with opposing circumferentially adjacent modules. The modules include longitudinally adjacent slide-and-lock radial elements which permit one-way sliding of the radial elements from a collapsed diameter to an expanded or deployed diameter, but inhibit radial recoil from the expanded diameter. The slide-and-lock elements may flex or bend; however, unlike stents described above that are compressed and expanded through flexing or bending of elements, no substantial plastic deformation of the elements may be necessary during expansion of the stent from a collapsed diameter to an expanded diameter.

Regardless of design, delivery typically includes inserting the stent through small lumens using a catheter and transporting it to the treatment site. Deployment includes expanding the stent to a larger diameter once it is at the desired location. Mechanical intervention with stents has reduced the rate of restenosis as compared to balloon angioplasty. Yet, restenosis remains a significant problem. When restenosis does occur in the stented segment, its treatment can be challenging, as clinical options are more limited than for those lesions that were treated solely with a balloon.

Stents are used not only for mechanical intervention but also as vehicles for providing biological therapy. Biological therapy uses medicated stents to locally administer a therapeutic substance. Effective concentrations at the treated site require systemic drug administration which often produces adverse or even toxic side effects. Local delivery is a preferred treatment method because it administers smaller total medication levels than systemic methods, but concentrates the drug at a specific site. Local delivery thus produces fewer side effects and achieves better results.

A medicated stent may be fabricated by coating the surface of a structure designed to provide patency with a polymeric carrier that includes an active or bioactive agent or drug. The structure itself may also serve as a carrier of an active agent or drug.

The stent must be able to satisfy a number of mechanical requirements. The stent must be capable of withstanding the structural loads, namely radial compressive forces, imposed on the stent as it supports the walls of a vessel. Therefore, a stent must possess adequate radial strength. Radial strength describes the external pressure that a stent is able to withstand without incurring clinically significant damage. Additionally, a stent should be sufficiently rigid to adequately maintain its size and shape throughout its service life despite the various forces that may come to bear on it, including the cyclic loading induced by the beating heart. For example, a radially directed force may tend to cause a stent to recoil inward. Furthermore, the stent should possess sufficient toughness or resistance to fracture from stress arising from crimping, expansion, and cyclic loading.

Some treatments with implantable medical devices require the presence of the device only for a limited period of time. Once treatment is complete, which may include structural tissue support and/or drug delivery, it may be desirable for the stent to be removed or disappear from the treatment location. One way of having a device disappear may be by fabricating the device in whole or in part from materials that erode or disintegrate through exposure to conditions within the body. Thus, erodible portions of the device can disappear or substantially disappear from the implant region after the treatment regimen is completed. After the process of disintegration has been completed, no portion of the device, or an erodible portion of the device will remain. In some embodiments, very negligible traces or residue may be left behind. Stents fabricated from biodegradable, bioabsorbable, and/or bioerodable materials such as bioabsorbable polymers can be designed to completely erode only after the clinical need for them has ended.

A similar manufacturing method is disclosed in US 2007/0207186.

However, there are potential shortcomings in the use of polymers as a material for implantable medical devices, such as in, for example, slide and lock stents. There is a need for manufacturing processes or material modifications for stents that addresses such shortcomings so that a polymeric stent can better meet the clinical and mechanical requirements of a stent.

### SUMMARY OF THE INVENTION

Various embodiments of the present invention include a method of making a stent comprising: stretching polymer sheet along an axis, wherein the stretching increases the strength, fracture toughness, and modulus of the polymer along the axis of stretching; and forming a tubular stent from the stretched sheet, the stent comprising a slide-and-lock mechanism, the slide and lock mechanism permitting the stent to move from a collapsed diameter to an expanded diameter and inhibiting radial recoil from the expanded diameter.

Additionnally disclosed is : a method of making a stent comprising: heating a polymer sheet to facilitate stretching of the sheet; stretching polymer sheet along an axis, wherein the stretching increases the strength, fracture toughness, and modulus of the polymer; actively cooling the deformed sheet to below a target temperature to stabilize the sheet at or close to a stretched state; and fabricating a stent from the deformed sheet after the cooling.

### BRIEF DESCRIPTION OF THE DRAWINGS

FIG. 1A shows a stent with a slide-and-lock design in a partially-expanded state.
FIG. 1B shows the stent of FIG. 1A in an expanded state.
FIG. 1C illustrates a slide-and-lock radial element of the stent in FIGs. 1A-B.
FIG. 2 depicts an x-y coordinate plane for illustrating the relationship between an axis of stretching and a cylindrical axis of a stent formed from a sheet.
FIG. 3A depicts stretching of a sheet with a tensile force.
FIG. 3B depicts an axial projection of a stent formed from the sheet stretched in FIG. 3A.

### DETAILED DESCRIPTION OF THE INVENTION

Various embodiments of the present invention relate to manufacture of stents from polymeric sheets. In particular, embodiments include methods of fabricating stents with slide-and-lock mechanisms. The fabrication methods are also applicable to making stents with other designs from sheets, such as, radially compressible/expandable scaffolding designs in which elements bend or flex with plastic deformation and jellyroll designs in which a sheet is rolled up upon itself with a high degree of overlap.

The embodiments include stretching or deforming a polymer sheet along at least one axis and forming a stent from the stretched or deformed sheet. The methods described herein are generally applicable to any polymeric implantable medical device. The methods can be applied to any tubular implantable medical devices that can be formed from a polymer sheet, such as self-expandable stents, balloon-expandable stents, and stent-grafts.

Expandable stents having and operating with slide-and-lock mechanisms or "slide-and-lock" stents include opposing circumferentially adjacent modules. The modules may include longitudinally adjacent slide-and-lock radial elements which permit one-way sliding of the radial elements from a collapsed diameter to an expanded/deployed diameter. The slide-and-lock radial elements can inhibit radial recoil from the expanded diameter. Slide-and-lock stent designs are described, for example, in U.S. Patent Pub. No. 20060136041. The radial elements are slidably interconnected with circumferentially adjacent radial elements in a manner in which the stent exhibits mono-directional radial expansion from a radially collapsed state to a radially expanded state, e.g., during deployment. The radial elements are configured to provide a ratcheting effect such that the stent is maintained (i.e., "locked-out") in the expanded diameter after deployment within the body passage.

The structures of slide-and-lock stents (e.g., radial elements) may flex or bend during expansion of the stent. However, unlike balloon expandable stents with compressible/expandable scaffolding, no substantial plastic deformation of the elements may be necessary during expansion of the stent from a collapsed diameter to an expanded diameter. Elements of this type may be referred to as "non-deforming elements." Accordingly, the term "non-deforming element" is intended to generally describe a structure that substantially maintains its original dimensions (i.e., length and width) during deployment of the stent.

A slide-and-lock stent can be formed from a flat sheet and rolled up to allow mating of circumferential adjacent radial elements. A flat sheet can be cut, machined, or etched to form the radial elements of the slide-and-lock mechanism or any other structures. For example, the elements or structures can be formed in a polymer sheet with a technique such as laser cutting or chemical etching.

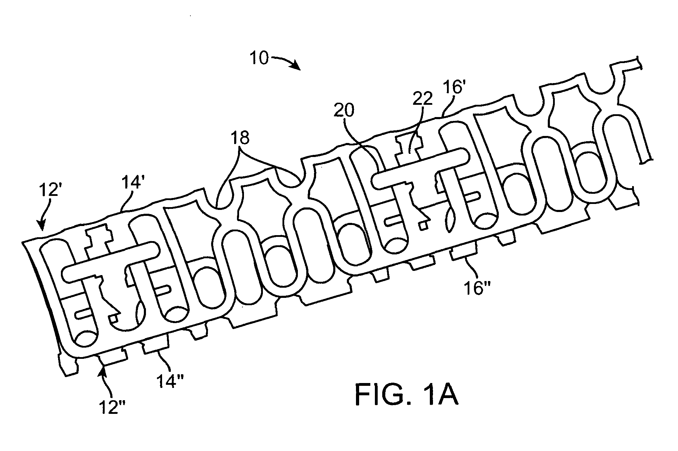

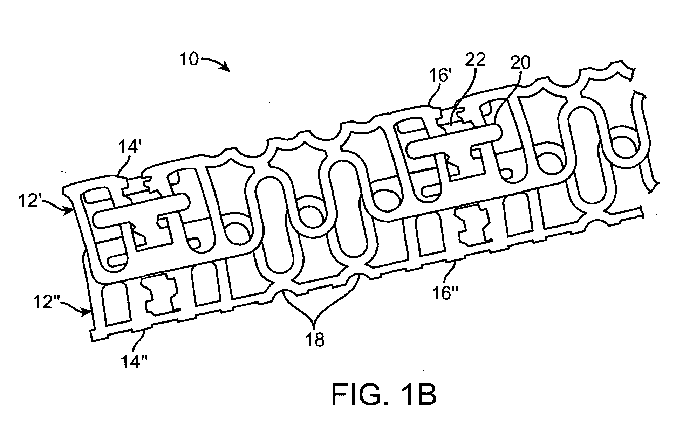

FIGs. 1A-B show partial views of an exemplary slide-and-lock stent 10. FIG. 1A shows stent 10 in a partially-expanded state and FIG. 1B shows stent 10 in an expanded state. The stent illustrated in FIGs. 1A-B is a slide-and-lock stent device that employs no actuating (that is, flexing, bending, and the like) elements to achieve expansion and lockout. A slide-and-lock stent can also employ an actuating catch member configured to deflect from a non-actuated position to an actuated position and back again as it passes the lockout tooth during expansion.

In FIGs. 1A-B, two circumferentially adjacent modules 12' and 12", each having longitudinally offset slide-and-lock radial elements, 14' and 16' in module 12', and 14" and 16" in module 12". Modules generally have at least two (2) slide-and-lock radial elements, at proximal and distal ends of the module. The slide-and-lock radial elements may be referred to as mechanism radial elements, because they include the slide-and-lock mechanisms that provide controlled deployment and resist radial compression. In embodiments of these modules, there may be between 2 and 8 slide-and-lock radial elements, or, between 2 and 4 slide-and-lock radial elements per module.

In some embodiments, such as that illustrated in FIGs. 1A-C, the longitudinally offset slide-and-lock radial elements within a module are separated and interconnected by one or more passive radial elements, such as the two (2) passive radial elements 18 shown in FIGs. 1A-B. These passive radial elements can be referred to as non-mechanism radial elements because they do not contribute to the slide-and-lock mechanism of radial expansion, like the slide-and-lock radial elements. In some embodiments, there are no passive radial elements. In other embodiments, there are from 1 to 8 passive radial elements disposed between each slide-and-lock radial element. These passive, non-mechanism radial elements can be engineered in many different geometric configurations to provide inter alia variable flexibility, variable radial strength, variable scaffolding (vessel wall coverage), and/or a safety catch to prevent over-expansion.

As can be seen in FIGs. 1A-B, a tab 20 on each slide-and-lock radial element (shown here on 16") is slidably engaged within a slot 22 in the circumferentially adjacent slide-and-lock radial element (shown here in 16'). The entire circumference of stent 10 may include from 1-8, 2-6, or 2-4 circumferentially adjacent radial elements.

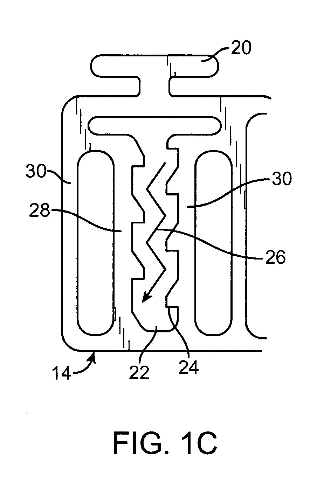

FIG. 1C illustrates, a slide-and-lock radial element 14 having a slot 22 with lockout teeth, catches or stops 24. When a tab 20 is slidably engaged within a slot 22 from a circumferentially adjacent slide-and-lock radial element, it can travel within the slot 22, thereby traveling through a defined travel path, as generally indicated by an arrow 26 in FIG. 1C. Stops 24 are circumferentially offset from one another and disposed on alternating proximal 28 and distal 30 sides or walls of the slot. The travel path may be disposed substantially in the circumferential axis as shown or the travel path may traverse both circumferential and longitudinal axes.

A slide-and-lock stent can also include deformable radial elements 14, for example, similar to those shown in FIG. 1A-B. A slide-and-lock radial element 14 can include a deformable region that deforms to allow expansion and/or contraction in the radial axis through material deformation. The deformable region can have a variety of material configurations, including for example, zigzag, U-shaped, serpentine, waves, undulating, and angled configurations, as well as changes in material cross-section so as to produce regions of deformability.

An implantable medical device can be made partially or completely from a biodegradable, bioabsorbable, or biostable polymer. A polymer for use in fabricating an implantable medical device can be biostable, bioabsorbable, biodegradable, or bioerodable. Biostable refers to polymers that are not biodegradable. The terms biodegradable, bioabsorbable, and bioerodable are used interchangeably and refer to polymers that are capable of being completely degraded and/or eroded when exposed to bodily fluids such as blood and can be gradually resorbed, absorbed, and/or eliminated by the body. The processes of breaking down and absorption of the polymer can be caused by, for example, hydrolysis and metabolic processes.

A stent made from a biodegradable polymer is intended to remain in the body for a duration of time until its intended function of, for example, maintaining vascular patency and/or drug delivery is accomplished. After the process of degradation, erosion, absorption, and/or resorption has been completed, no portion of the biodegradable stent, or a biodegradable portion of the stent will remain. In some embodiments, very negligible traces or residue may be left behind.

The duration of a treatment period depends on the bodily disorder that is being treated. In treatments of coronary heart disease involving use of stents in diseased vessels, the duration can be in a range from about a few months to a few years. However, the duration is typically up to about six months, twelve months, eighteen months, or two years. In some situations, the treatment period can extend beyond two years.

As indicated above, a stent has certain mechanical requirements such as high radial strength, high modulus, and high fracture toughness. A stent that meets such requirements greatly facilitates the delivery, deployment, and treatment of a diseased vessel. A polymeric stent with inadequate mechanical properties can result in mechanical failure or recoil inward after implantation into a vessel.

With respect to radial strength, the strength to weight ratio of polymers is smaller than that of metals. To compensate for this, a polymeric stent can require significantly thicker struts than a metallic stent, which results in an undesirably large profile.

Additionally, polymers that are rigid at conditions within the human body may also have low fracture toughness since they may exhibit a brittle fracture mechanism. For example, these include polymers that have a glass transition temperature (Tg) above human body temperature (Tbody), which is approximately 37 °C. Such polymers may exhibit little or no plastic deformation prior to failure. It is important for a stent to be resistant to fracture throughout the range of use of a stent, i.e., crimping, delivery, deployment, and during a desired treatment period.

One way of addressing the strengths deficiency that some polymers have is to fabricate a stent from a deformed polymer construct. Deforming polymers tends to increase the strength and stiffness along the direction of deformation. We have observed that deformation also tends to increase fracture toughness of polymer constructs and stents. Without being limited by theory, the increased strength and modulus are due to alignment of polymer chains or a preferred polymer chain orientation along the axis or direction of deformation.

Certain embodiments of the invention include fabricating a slide-and-lock stent from a stretched or deformed polymer sheet. In such embodiments, a polymer sheet may be stretched along one or more axes. A stent may be formed from the stretched sheet, or more generally, from a rectangular portion of the stretched sheet. The stretched sheet or portion thereof can be cut or machined to form opposing circumferentially adjacent slide-and-lock modules. Upon forming the slide-and-lock modules, the sheet can be rolled up in the form of a tube and opposing modules mated to secure the stent in a reduced or delivery diameter.

In some embodiments, the sheet may be plastically deformed along the axis of stretching. Alternatively, the sheet may be stretched elastically.

Additionally, the tube may be formed with a cylindrical axis of the tube parallel, perpendicular, or at an angle between parallel and perpendicular to the axis of stretching. In particular, the radial strength of the stent may be maximized when a stent is formed so that the axis of stretching is perpendicular or substantial perpendicular to the cylindrical axis.

The degree of stretching of the sheet may be quantified by strain (ε), L/L₀, where L₀ is the original length and L is the stretched length. Alternatively, the degree of stretching can be expressed as the percent stretching or (ε-1) x 100%. In exemplary embodiments, the percent stretching can be 0-100%, 100-300%, 300-500%, or greater than 500%.

In further embodiments, the stent can be formed from a sheet stretched along at least one additional axis. In particular, the stent can be formed from a sheet stretched along two axes so that the sheet and the stent formed from the sheet have biaxial orientation. The two different axes may be at any angle relative to one another. In a particular embodiment, the two axes of stretching are perpendicular. In such an embodiment, the stent may be formed so that one axis of stretching is parallel and one axis is perpendicular to the cylindrical axis of the stent. In addition, the degree of stretching or strain along the axes can be the same or different.

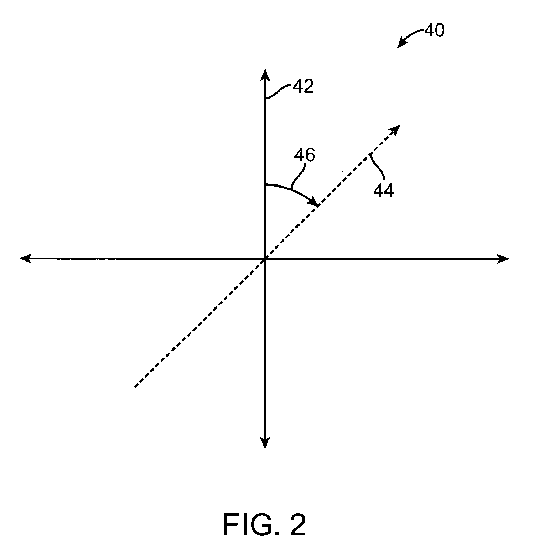

FIG. 2 depicts an x-y coordinate plane 40 for illustrating the relationship between an axis of stretching 42 and a cylindrical axis 44 of a stent formed from a sheet. For example, a stent may be formed from a stretched sheet with an angle 46 between the axis of stretching and cylindrical axis.

In one embodiment, a sheet or film of polymer may be stretched along an axis or two axes using a tenter. In a tenter, stretching is generally performed inside of a box. The sheet is grasped on either side by tenterhooks that exert a tensile force or drawing tension along at least one axis. The temperature inside of the box may be controlled.

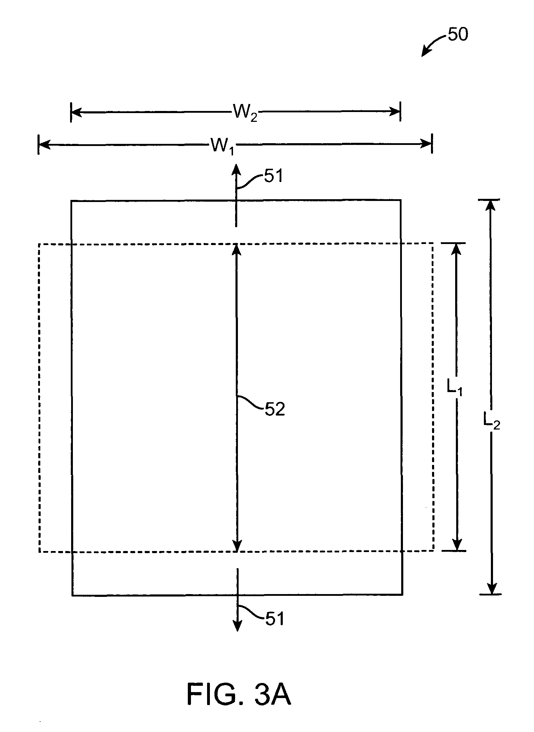

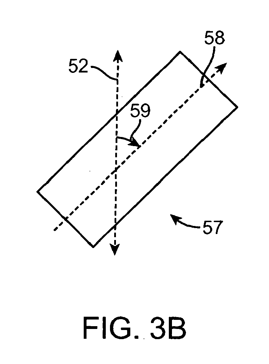

FIG. 3A depicts stretching of a sheet 50 with a tensile force 51. Sheet 50 may be stretched or elongated along an axis 52 parallel to tensile force 51. Tensile force 51 causes sheet 50 to stretch along axis of stretching 52. Sheet 50 has a length L₁ prior to stretching and a length L₂ after stretching. Tensile force 51 and the resultant stretching of sheet 50 may cause a decrease in a width of sheet 50 from W₁ to W₂. FIG. 3B depicts an axial projection of a stent 57 formed from sheet 50 after stretching. Stent 57 has a cylindrical axis 58 which is at an angle 59 relative to axis of stretching 52.

In an embodiment, the tensile force for stretching a sheet may be selected to achieve a desired degree of stretching. The tensile force may be constant or variable with time. In an embodiment, the tensile force may be adjusted to cause either a constant rate of stretching or a constant strain rate.

Various polymers can be used to fabricate a stent as described herein. These include semicrystalline polymers and amorphous polymers. Additionally, the stent can be made from an amorphous partially crosslinked polymer. The polymer sheet may be formed from a crosslinked polymer with low crosslinking degree prior to the stretching step. Alternatively, the polymer sheet can be crosslinked during or after stretching. The polymer and processing conditions are selected to stabilize the polymer orientation induced by the stretching and to enhance or optimize the fracture toughness.

Generally, the fabrication method includes heating a polymer sheet above an ambient temperature to facilitate or allow deformation, deforming the heated sheet, and cooling the deformed sheet to stabilize the oriented polymer structure. In one embodiment, the sheet is made of a semicrystalline polymer with a Tg above Tbody. The lack of mobility of the polymer chains below Tg stabilizes the polymer structure after processing and upon implantation in a patient. In order ensure sufficient stability of the stent at conditions within a human body, the Tg should be at least 10, 20, or 30 °C greater than Tbody.

In another embodiment, the sheet is an amorphous polymer with a Tg above Tbody and the induced polymer structure is also stabilized due to lack of mobility of polymer chains below Tg. In other embodiments, when the stent is made from a crosslinked polymer, the crosslinks stabilize the polymer structure after processing and implantation. The crosslinks may be distributed uniformly throughout the polymer. In some embodiments of a crosslinked polymer, the Tg of the polymer is greater than Tbody and both the lack of mobility of the chains and the crosslinks stabilize the induced orientation of the polymer.

Exemplary semicrystalline polymers include poly(L-lactide), polyglycolide (PGA), polymandelide (PM), poly(4-hydroxy butyrate) (PHB), poly(L-lactide-co-glycolide) (PLGA), poly(L-lactide-co-caprolactone). Exemplary amorphous polymers include poly(DL-lactide) (PDLLA), and poly(DL-lactide-co-caprolactone). PLLA, PM, PGA, PDLLA, and PLGA have Tg's above Tbody. Additionally, each of the tyrosine-derived polycarbonates referred to below, poly(DTE carbonate), poly(DTB carbonate), poly(DTH carbonate), poly(DTO carbonate), and poly(DTBzl carbonate), is amorphous with a Tg above Tbody. Polymeric Materials Encylopedia, ed. by J. Salamone, CRC Press, Vol. 9 p. 7280 (1996).

In the embodiments of a sheet polymer having a Tg greater than Tbody, the polymer sheet is stretched at a temperature above the glass transition temperature (Tg), but less than the melting temperature, Tm, of the sheet polymer. In general, when the temperature of a polymer is increased above its Tg, the polymer transforms from a brittle vitreous state to a solid deformable or ductile state. In this ductile state, polymer chains have sufficient freedom of movement to reorient preferentially along a direction of stretching or deformation. A target temperature for deformation may be at least 5, 10, 20, or 30°C above Tg, or greater than 30°C above Tg. The polymer sheet can be heated in a variety of ways. For example, a warm gas (e.g., air, nitrogen, argon, etc.) can be blown on the sheet with a nozzle, the sheet can be positioned between heated plates, or heated in an oven.

It is generally preferable to heat the sheet uniformly or nearly uniformly to obtain spatially uniform mechanical properties in the sheet. The heating methods mentioned above can be adapted to perform uniform heating of the polymer sheet. For example, uniform heating can be performed by an elongate nozzle blowing warm gas. The elongate nozzle may extend along the length of one dimension of the sheet and rapidly translates above the sheet surface along the other dimension. Additionally, a heated plate adjacent to the sheet surface or heating in an oven can provide uniform heating.

The sheet may be heated to the target temperature prior to applying a tension that elongates the sheet. The heating may continue during and after the deformation. In some embodiments, the sheet can be held under tension at the target elongation after stretching while maintaining the target temperature or another temperature.

As indicated above, the sheet is cooled to below Tg after stretching to a target elongation. Cooling below Tg stabilizes the induced orientation that is believed to give rise to the improved mechanical properties. The sheet is typically cooled to room or ambient temperature (i.e., 20-30°C) prior to further processing steps, such as laser machining. In some embodiments, the stretched sheet may be allowed to cool passively under ambient conditions.

Alternatively, the stretched sheet may be actively cooled by exposing the sheet to a cold medium that is below ambient temperature. For example, a nozzle can blow a cold gas on the sheet. The cold gas can be less than 10, 0, or -10°C. In some cases, the stretched sheet is rapidly cooled or quenched to below Tg. For example, the stretched sheet can be cooled to below Tg in less than 5 to 30 seconds. Quenching or quickly cooling the stretched sheet can reduce or prevent loss of induced polymer orientation.

Furthermore, for a sheet made from a semicrystalline polymer, the thermal and deformation history are particularly important for enhancing the fracture toughness. Embodiments include deformation of a polymer sheet in which the thermal and deformation history are controlled to enhance the fracture toughness of the expanded sheet and stent formed therefrom.

Generally, when semicrystalline polymers crystallize, two separate events occur. The first event is the formation of nuclei in an amorphous polymer matrix. The second event is growth of a crystallite around these nuclei. The overall rate of crystallization of the polymer is dependent, therefore, on the equilibrium concentration of nuclei in the polymer matrix, and on the rate of growth of crystallites around these nuclei.

Semicrystalline polymers can contain both amorphous and crystalline domains at temperatures below the melting point. In general, crystallization tends to occur in a polymer at temperatures between Tg and Tm of the polymer. Both the nucleation and crystallite growth rate increase from zero at Tg, reach a maximum, and then decrease to zero at Tm. At temperatures above Tg, but far below Tm, nucleation is substantially favored over crystallite growth, since the latter process requires much more extensive chain mobility. However, at higher temperatures above Tg, but below Tm, crystallite growth is favored over nucleation. A consequence of the behavior is that at high temperatures there are relatively few, large crystallites formed, while at low temperatures, there are relatively more numerous, smaller crystallites formed.

Most semicrystalline polymers crystallize slowly from the quiescent state, but orders of magnitude faster when the material is subjected to strong deformations. These deformations are characteristic of processing above the Tg, but below the Tm. Such crystallization is referred to in physics as strain-induced (or flow-induced) crystallization. Liedauer, et al., Int. Polym. Proc. 8, 236-244 (1993); Kumaraswamy, et al., Macromolecules 35, 1762-1769 (2002).

There are several parameters related to crystallinity that influence the fracture toughness of a polymer. Fracture toughness tends to be directly proportional crystallite density and inversely proportional to crystallite size. As a result, fracture toughness is particularly enhanced or improved when a polymer has a relatively high crystallite density with crystallites that are relatively small in size. For example, a crystallite size (crystal lamellar thickness) of less than about 50 nm is desirable. Thus, since nucleation rate is faster than crystallite growth rate at lower temperature close to Tg, it is preferable for the temperature of the sheet to be in this range during stretching.

Additionally, the degree of crystallinity also affects the fracture toughness. A crystallinity that is too high can result in undesirable decreased fracture toughness and brittle behavior. The crystallinity obtained depends upon the temperature and the time that the sheet is in the crystallization temperature range, i.e., between Tg and Tm.

There are various processing variables of the deformation process, which may influence the fracture toughness and other properties of a deformed sheet. These include the deformation temperature, the time the sheet is heated until it is deformed at the deformation temperature (heating time), the deformation time or the time to stretch the sheet from an initial state to the final stretched state, the time delay between the reaching the final stretched state of the sheet and the start of active cooling (cooling delay), and the duration of active cooling of the sheet (active cooling time), and the time for the active cooling to cool the sheet to below Tg (Tg cooling time). In a typical embodiment, the active cooling time may be higher than the Tg cooling time.

"Active cooling" refers to cooling the sheet through exposure of the sheet to conditions below ambient temperature that results in a decrease in temperature of the sheet. Passive cooling includes to allowing the sheet to cool only through the exposure of the sheet to ambient conditions. During the cooling delay, the sheet may be cooled passively or experience a decrease in temperature through exposure of the sheet to ambient temperature or above ambient temperature. Alternatively, heating of the sheet may continue during some or all of the cooling delay.

The fracture toughness of a sheet is believed to be dependent upon the parameters of deformation temperature, deformation time, heating time, cooling delay, Tg cooling time, and the active cooling time. The parameters may be adjusted to obtain a desired or optimum fracture toughness, modulus, and radial strength of the deformed sheet. In some embodiments, values of the parameters that achieve a high fracture toughness may be determined by measuring and evaluating properties related to fracture toughness for various values of the parameters. For example, the elongation at break of sheet specimens or the number of cracks in a stent made from the sheet after deployment can be measured for various values of the parameters.

The deformation temperature refers to the temperature of the sheet when it is stretched. In some embodiments, the deformation temperature can vary during the stretching. A higher fracture toughness for a semicrystalline polymer may be achieved by a relatively low deformation temperature, for example, a temperature that is above and close to Tg. In exemplary embodiments, a deformation temperature or range can be less 2 °C, 2-5 °C, 5-10 °C, or greater than 10 °C above a Tg of the polymer of the sheet. Alternatively, the deformation temperature or range can be between Tg + 0.1 x (Tm - Tg) and Tg + 0.5 x (Tm - Tg). In addition, in some embodiments, a higher fracture toughness may be achieved by a rapid heating time, short cooling delay, and fast Tg cooling time. For example, for a sheet that is made of or consists essentially of PLLA, the deformation temperature can be between 65 and 100 °C.

As indicated above, a relatively rapid heating time may facilitate making a deformed sheet with a high fracture toughness. It is believed that the rapid heating time reduces the amount of time the sheet temperature is a crystallization range in which nucleation and growth occurs. If the heating is too slow, the increase in crystallinity from the heating coupled with the strain-induced crystallization from the deformation can result in a sheet that is brittle with low fracture toughness.

Typically, the polymer sheet is heated from Tambient. In exemplary embodiments, a PLLA polymer sheet is heated from Tambient to a deformation temperature in less than 10 s, between 10-20 s, between 20-40 s, 40-60 s, or greater than 60 s.

In certain embodiments, the cooling delay is reduced or minimized to reduce or eliminate a further increase crystallinity after deformation and to stabilize or freeze the induced polymer chain orientation. In some embodiments, the cooling of the deformed sheet starts immediately or slightly after (less than 1 sec) the sheet has reached a final stretched state. In other embodiments, a cooling delay is selected to allow a further increase in crystallinity that improves mechanical properties. Also, a cooling delay help may relieve internal stress in the polymer which results in dimensional instability (i.e., a distortion in shape of sheet). An exemplary cooling delay may be less than 2 s, 5 s, 10 s, or greater than 10 s.

As indicated above, a stretched sheet for use in fabricating a stent can be made from a crosslinked polymer. As used herein, crosslinks refer generally to chemical covalent bonds that link one polymer chain to another. A crosslinked polymer includes crosslinks throughout the bulk of a polymer. When polymer chains are linked together by crosslinks, they lose some of their ability to move as individual polymer chains. Thus, the crosslinks inhibit or prevent relaxation of the oriented structure of the polymer.

The degree of crosslinking may be characterized by crosslink or crosslinking density. The crosslink density can be expressed as the average molecular weight (number average or weight average) between crosslink sites (Mc). Alternatively, the crosslink density can be expressed as the mole fraction of monomer units which are crosslink points (Xc). An Introduction to Plastics, Hans-Georg Elias 2nd ed. Wiley (2003). Crosslink density can be determined by known methods such as dynamical mechanical analysis (DMA).

In certain embodiments, a polymer sheet can be formed from a precursor polymer that is uncrosslinked. An uncrosslinked polymer can correspond to polymer that has not been subjected or exposed to conditions (e.g., heat or radiation) or compounds (e.g., crosslinking agents) other than those encountered in processing or forming the polymer sheet (e.g., extrusion). The precursor polymer can be a semicrystalline or amorphous polymer. Crosslinking of the precursor polymer may be induced during stretching of the polymer sheet, after stretching of the polymer sheet to a target strain, or both. After the sheet is stretched, the crosslinks inhibit or prevent relaxation of the oriented polymer structure induced by stretching. In such embodiments, the polymer sheet can be heated and cooled as described above.

Crosslinks can be formed by chemical reactions that are initiated by heat, pressure, or radiation. The radiation can include, but is not limited to, electron beam, gamma, or UV light. In some embodiments, a polymer sheet may be exposed to radiation to induce crosslinking during stretching, upon reaching a target strain, or both. The crosslinking induced by radiation can be caused by or facilitated by a crosslinking agent. Radiation crosslinking of polylactic acids with crosslinking agents have been described, for example, in Mitomo, Hiroshi et al., Polymer 46 4695-4703 (2005) and Quynh, Tran et al., European Polymer Journal 43 1779-1785 (2007). The radiation dose, type and mole or weight percent of a crosslinking agent can also influence the crosslink density. The radiation does is directly proportional to the crosslink density.

In other embodiments, the polymer sheet may be formed from a polymer that already has crosslinks prior to stretching. For example, the polymer sheet prior to stretching can have a crosslink degree of less than 0.5%, 1%, or 2%. In these embodiments, the crosslinked polymer sheet is stretched, which induces orientation of the crosslinked polymer chains. The crosslinks may then inhibit or prevent relaxation of induced orientation. In some embodiments, further crosslinking can be induced during stretching, after stretching to a target strain, or both. In such embodiments, the polymer sheet can be heated and cooled as described above.

Mechanical properties, such as strength, modulus, and fracture toughness depend upon the degree of crosslinking or crosslink density. As the crosslinking increases, the polymer becomes stiffer. However, as the crosslink degree increases, a material can become brittle. In exemplary embodiments, the crosslink degree is less than 0.5%, 1%, or less than 2%.

In further embodiments, nanoparticles can be dispersed in the sheet to increase the fracture toughness of a stent made from the sheet. As used herein, nanoparticles refer to particles with a size in a range less than 1 micron. Such particles can have a size range less than 800 nm, less than 500 nm, less than 200 nm, or less then 100 nm. The particles can be mixed or dispersed in the polymer during the making of the sheet, for example, during extrusion. The particles can be nonerodible or erodible upon exposure to bodily fluids.

Exemplary classes of material include bioceramic particles, metallic particles or fibers, carbon nanotubes or nanofibers, and quantum dots. Exemplary bioceramic particles include hydroxylapatite and calcium phosphate. The metallic particles can include magnesium, iron, and tungsten. A "quantum dot" refers to a semiconductor whose excitons are confined in all three spatial dimensions.

### Definitions:

For the purposes of the present invention, the following terms and definitions apply:
The "glass transition temperature," Tg, is the temperature at which the amorphous domains of a polymer change from a brittle vitreous state to a solid deformable or ductile state at atmospheric pressure. In other words, the Tg corresponds to the temperature where the onset of segmental motion in the chains of the polymer occurs. When an amorphous or semicrystalline polymer is exposed to an increasing temperature, the coefficient of expansion and the heat capacity of the polymer both increase as the temperature is raised, indicating increased molecular motion. As the temperature is raised the actual molecular volume in the sample remains constant, and so a higher coefficient of expansion points to an increase in free volume associated with the system and therefore increased freedom for the molecules to move. The increasing heat capacity corresponds to an increase in heat dissipation through movement. Tg of a given polymer can be dependent on the heating rate and can be influenced by the thermal history of the polymer. Furthermore, the chemical structure of the polymer heavily influences the glass transition by affecting mobility.

"Stress" refers to force per unit area, as in the force acting through a small area within a plane. Stress can be divided into components, normal and parallel to the plane, called normal stress and shear stress, respectively. Tensile stress, for example, is a normal component of stress applied that leads to expansion (increase in length). In addition, compressive stress is a normal component of stress applied to materials resulting in their compaction (decrease in length). Stress may result in deformation of a material, which refers to a change in length. "Expansion" or "compression" may be defined as the increase or decrease in length of a sample of material when the sample is subjected to stress.

"Strain" refers to the amount of expansion or compression that occurs in a material at a given stress or load. Strain may be expressed as a fraction or percentage of the original length, i.e., the change in length divided by the original length. Strain, therefore, is positive for expansion and negative for compression.

"Strength" refers to the maximum stress along an axis which a material will withstand prior to fracture. The ultimate strength is calculated from the maximum load applied during the test divided by the original cross-sectional area.

"Modulus" may be defined as the ratio of a component of stress or force per unit area applied to a material divided by the strain along an axis of applied force that results from the applied force. For example, a material has both a tensile and a compressive modulus.

The tensile stress on a material may be increased until it reaches a "tensile strength" which refers to the maximum tensile stress which a material will withstand prior to fracture. The ultimate tensile strength is calculated from the maximum load applied during a test divided by the original cross-sectional area. Similarly, "compressive strength" is the capacity of a material to withstand axially directed pushing forces. When the limit of compressive strength is reached, a material is crushed.

"Toughness" is the amount of energy absorbed prior to fracture, or equivalently, the amount of work required to fracture a material. One measure of toughness is the area under a stress-strain curve from zero strain to the strain at fracture. The units of toughness in this case are in energy per unit volume of material. See, e.g., L. H. Van Vlack, "Elements of Materials Science and Engineering," pp. 270-271, Addison-Wesley (Reading, PA, 1989).

The underlying structure or substrate of an implantable medical device, such as a stent can be completely or at least in part made from a biodegradable polymer or combination of biodegradable polymers, a biostable polymer or combination of biostable polymers, or a combination of biodegradable and biostable polymers. Additionally, a polymer-based coating for a surface of a device can be a biodegradable polymer or combination of biodegradable polymers, a biostable polymer or combination of biostable polymers, or a combination of biodegradable and biostable polymers.

It is understood that after the process of degradation, erosion, absorption, and/or resorption has been completed, no part of the stent will remain or in the case of coating applications on a biostable scaffolding, no polymer will remain on the device. In some embodiments, very negligible traces or residue may be left behind. For stents made from a biodegradable polymer, the stent is intended to remain in the body for a duration of time until its intended function of, for example, maintaining vascular patency and/or drug delivery is accomplished.

Representative examples of polymers that may be used to fabricate an implantable medical device include, but are not limited to, poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(glycolic acid), poly(glycolide), poly(L-lactic acid), poly(L-lactide), poly(D,L-lactic acid), poly(D,L-lactide), poly(caprolactone), poly(trimethylene carbonate), polyester amide, poly(glycolic acid-co-trimethylene carbonate), co-poly(ether-esters) (e.g. PEO/PLA), polyphosphazenes, biomolecules (such as fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (such as polyvinyl chloride), polyvinyl ethers (such as polyvinyl methyl ether), polyvinylidene halides (such as polyvinylidene chloride), polyacrylonitrile, polyvinyl ketones, polyvinyl aromatics (such as polystyrene), polyvinyl esters (such as polyvinyl acetate), acrylonitrile-styrene copolymers, ABS resins, polyamides (such as Nylon 66 and polycaprolactam), polycarbonates, polyoxymethylenes, polyimides, polyethers, polyurethanes, rayon, rayon-triacetate, cellulose, cellulose acetate, cellulose butyrate, cellulose acetate butyrate, cellophane, cellulose nitrate, cellulose propionate, cellulose ethers, and carboxymethyl cellulose. Another type of polymer based on poly(lactic acid) that can be used includes graft copolymers, and block copolymers, such as AB block-copolymers ("diblock-copolymers") or ABA block-copolymers ("triblock-copolymers"), or mixtures thereof.

Poly(amino acids) are a broad class of polymers that can be degraded by cellular or enzymatic mechanisms to their respective amino acids. Such polymers suffer from the disadvantage that they are difficult to process due to a relatively low decomposition temperature. However, a class of poly(amino acids) has been developed in which a-L-amino acids are linked by nonamide bonds such as ester, carbonate or iminocarbonate linkages. These polymeric systems are referred to as 'pseudo'-poly(amino acids). The presence of nonamide bonds in the polymeric backbone greatly improves the physicomechanical properties and processibility of pseudo-poly-(amino acids). Furthermore, naturally occurring amino acids as the polymeric building blocks can reduce the potential toxicity of degradation products released into the biological system.

Tyrosine-derived polycarbonates, another representative class of polymer for fabricating an implantable medical device, represent a specific example of pseudo-poly(amino acids). These degradable polymers are derived from the polymerization of desaminotyrosyl-tyrosine alkyl esters. J. of Appl. Polymer Sci., Vol. 63, 11, pp. 1467-1479. In the synthesis of tyrosine-derived polycarbonates, L-tyrosine and its natural metabolite desaminotyrosine [3-(4*-hydroxphenyl) propionic acid] are used as building blocks to form desaminotyrosyl-tyrosine alkyl esters. These diphenolic monomers are then polymerized to provide tyrosine-derived polyiminocarbonates, polycarbonates, and polyarylates. In particular, tyrosine-derived polycarbonates are synthesized by polymerizing tyrosine in the presence of phosgene or bis(chloromethyl) carbonate triphosgene. Adv Biochem Engin/Biotechnol (2006) 102: 59.

A representative structure of a tyrosine-derived polycarbonate is: 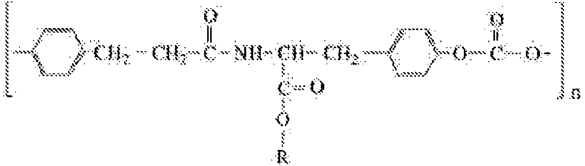 When R is a hydrogen, the repeat unit is desamino-tyrosyl-tyrosine, referred to as "DT." The pendent group (R) of the polycarbonates can also be, for example, ethyl, butyl, hexyl, octyl, and benzyl esters. The corresponding polymers are referred to as poly(DTE carbonate), poly(DTB carbonate), poly(DTH carbonate), poly(DTO carbonate), and poly(DTBzl carbonate), respectively. The ethyl pendent group may be preferred at least for the reason that the pendent groups are not biodegradable and a shorter pendent group is more easily and safely eliminated by the body.

The physicochemical properties of these polymers can be tailored by varying the pendant alkyl ester chain. These properties include the strength, stiffness, or modulus, Tg, and hydrophobicity.

Tyrosine-derived polycarbonates have been found to have strengths between 50-70 Mpa and stiffness (i.e., modulus) of 1-2 GPa with poly(DTE carbonate) being the strongest and stiffest. Medical Plastics: Degradation Resistance and Failure Analysis, Robert. C. Portnoy, William Andrews, Inc. (1998). Increasing the length of the pendent chain tends to decrease the strength and stiffness.

Poly(DTE carbonate) is stronger and stiffer than polycaprolactone, polydioxanone, and polyorthoesters. However, poly(DTE carbonate) is less stiff than PLLA (2.4-10 GPa) or polyglycolide (6.5 GPa). *Ibid* Tyrosine-derived polycarbonates have comparable strengths to PLLA (60-70 MPA) and PGA (60-80 MPa). Medical Device Manufacturing & Technology 2005.

The Tg of tyrosine-derived polycarbonates decreases from 93 °C to 52 °C when the length of the pendent carbon chain increases from two to eight carbon atoms. *Ibid.* The decomposition temperatures of the series are independent of the pendent chain length and exceed 293 °C. *Ibid.* The wide gap between the Tg and thermal decomposition temperatures makes these polymers readily processible by conventional polymer processing techniques including extrusion, compression molding, and injection molding. The polymer also is expected to show stability during sterilization due to the presence of aromatic groups in its backbone.

## Claims

1. A method of making a stent (10) comprising:
stretching a polymer sheet (50) along an axis (52), wherein the stretching increases the strength, fracture toughness, and modulus of the polymer; and
forming a tubular stent (57) from the stretched sheet, the stent comprising a slide-and-lock mechanism, the slide and lock mechanism permitting the stent to move from a collapsed diameter to an expanded diameter and inhibiting radial recoil from the expanded diameter.

2. The method of claim 1, wherein the polymer sheet is made from a biodegradable polyester selected from the group consisting of PLLA and PLGA.

3. The method of claim 1, wherein the polymer sheet comprises or is made from a tyrosine-derived polycarbonate.

4. The method of claim 1, wherein the slide and lock mechanism comprises circumferentially adjacent modules (12', 12"), the modules including longitudinally adjacent slide-and-lock radial elements (14', 16'; 14", 16") which permit one-way sliding of the radial elements from the collapsed diameter to the expanded diameter and inhibit radial recoil from the expanded diameter.

5. The method of Claim 1, further comprising stretching the sheet along a second axis of stretching to provide biaxial orientation to the sheet, wherein a cylindrical axis of the tube is parallel, perpendicular, or at an angle between parallel and perpendicular to the second axis of stretching.

6. The method of Claim 5, wherein the axis of stretching is perpendicular to the cylindrical axis and the second axis of stretching is parallel to the cylindrical axis.

7. The method of Claim 1, wherein the sheet consists essentially of a bioabsorbable polymer.

8. The method of Claim 1, wherein the slide and lock mechanism is formed by laser cutting the stretched sheet.

9. The method of Claim 1, wherein the stretching is performed at a temperature greater than or equal to a glass transition temperature (Tg) of the polymer and less than a melting temperature (Tm) of the polymer.

10. The method of Claim 1, wherein the polymer is semicrystalline or amorphous with a glass transition temperature (Tg) at least 10 °C above human body temperature (Tbody).

11. The method of Claim 10, further comprising heating the polymer sheet uniformly or substantially uniformly to a temperature at least 5 °C above the glass transition temperature (Tg) of the polymer and quenching the stretched sheet to a temperature below the glass transition temperature (Tg) after the stretching, wherein the reduction in temperature inhibits or prevents relaxation of polymer chain orientation induced by the stretching.

12. The method of Claim 1, wherein the polymer sheet is crosslinked prior to the stretching, wherein the crosslinks inhibit or prevent relaxation of the polymer chain orientation induced by the stretching, the crosslink degree being less than 5%.

13. The method of Claim 1, further comprising inducing crosslinking of the polymer sheet during or after the stretching, wherein the crosslinking inhibits or prevents relaxation of polymer chain orientation induced by the stretching.

14. The method of Claim 12, wherein the polymer is semicrystalline or amorphous and has a glass transition temperature (Tg) greater than human body temperature (Tbody).

15. The method of Claim 1, wherein the polymer sheet comprises nanoparticles dispersed in the polymer.

## Patentansprüche

1. Verfahren zur Herstellung eines Stents (10) umfassend:
das Strecken eines Polymerblattes (50) entlang einer Achse (52), wobei das Strecken die Festigkeit, die Bruchzähigkeit und den Modul des Polymers steigert; und
das Ausbilden eines rohrförmigen Stents (57) ausgehend vom gestreckten Blatt, der Stent umfassend eine Einrichtung zum Schieben und Arretieren (slide-and-lock), wobei die Einrichtung zum Schieben und Arretieren den Durchgang des Stents von einem kollabierten Durchmesser zu einem gestreckten Durchmesser ermöglicht und den radialen Rückstoß aus dem gestreckten Durchmesser verhindert.

2. Verfahren nach Anspruch 1, wobei das Polymerblatt aus einem biologisch abbaubaren Polyester hergestellt ist, der ausgewählt aus der Gruppe bestehend aus PLLA und PLGA ist.

3. Verfahren nach Anspruch 1, wobei das Polymerblatt ein aus Tyrosin abgeleitetes Polycarbonat enthält oder daraus hergestellt ist.

4. Verfahren nach Anspruch 1, wobei die Einrichtung zum Schieben und Arretieren umlaufend angrenzende Module (12', 12") umfasst, wobei die Module in Längsrichtung angrenzende radiale Einsätze zum Schieben und Arretieren umfasst (14', 16'; 14", 16"), die das Einweg-Schieben der radialen Einsätze aus dem kollabierten Durchmesser bis zum gestreckten Durchmesser ermöglichen und den radialen Rückstoß aus dem gestreckten Durchmesser verhindern.

5. Verfahren nach Anspruch 1, weiterhin umfassend das Strecken des Blattes entlang einer zweiten Streckungsachse um das Blatt biaxial auszurichten, wobei eine zylindrische Achse des Rohrs parallel, senkrecht, oder mit einem Winkel zwischen parallel und senkrecht bezüglich auf die zweite Achse ausgerichtet ist.

6. Verfahren nach Anspruch 5, wobei die Streckungsachse senkrecht zur zylindrischen Achse ist und die zweite Streckungsachse parallel zur zylindrischen Achse ist.

7. Verfahren nach Anspruch 1, wobei das Blatt im Wesentlichen aus einem bioresorbierbaren Polymer besteht.

8. Verfahren nach Anspruch 1, wobei die Einrichtung zum Schieben und Arretieren durch Laserschnitt des gestreckten Blattes ausgebildet wird.

9. Verfahren nach Anspruch 1, wobei das Strecken bei einer Temperatur ausgeführt wird, die der Glasübergangstemperatur (Tg) des Polymers entspricht oder höher ist und niedriger als eine Schmelztemperatur (Tm) des Polymers ist.

10. Verfahren nach Anspruch 1, wobei das Polymer halbkristallin oder amorph ist und eine Glasübergangstemperatur (Tg) aufweist, die mindestens 10°C höher als die menschliche Körpertemperatur (Tbody) ist.

11. Verfahren nach Anspruch 10, weiterhin umfassend das gleichmäßige oder im Wesentlichen gleichmäßige Erwärmen des Polymerblattes auf eine Temperatur, die mindestens 5 °C höher als die Glasübergangstemperatur (Tg) des Polymers ist und das Abkühlen des gestreckten Blattes nach dem Strecken auf eine Temperatur unterhalb der Glasübergangstemperatur (Tg), wobei die Temperatursenkung die Lockerung der Ausrichtung der Polymerkette, die durch die Streckung veranlasst wird, hemmt oder verhindert.

12. Verfahren nach Anspruch 1, wobei das Polymerblatt vor der Streckung quervernetzt ist, wobei die Quervernetzung die Lockerung der Ausrichtung der Polymerkette, die durch die Streckung veranlasst wird, hemmt oder verhindert, wobei der Quervernetzungsgrad bei unter 5% liegt.

13. Verfahren nach Anspruch 1, bei dem weiterhin die Quervernetzung des Polymerblattes während oder nach der Streckung veranlasst wird, wobei die Quervernetzung die Lockerung der Ausrichtung der Polymerkette, die durch die Streckung veranlasst wird, hemmt oder verhindert.

14. Verfahren nach Anspruch 12, wobei das Polymer halbkristallin oder amorph ist und eine Glasübergangstemperatur (Tg) höher als die menschliche Körpertemperatur (Tbody) aufweist.

15. Verfahren nach Anspruch 1, wobei das Polymerblatt Nanopartikeln umfasst, die im Polymer dispergiert sind.

## Revendications

1. Procédé de fabrication d'une endoprothèse vasculaire (10) comprenant:
étirer une feuille (50) de polymère le long d'un axe (52), dans lequel l'étirement augmente la solidité, la ténacité, et le module du polymère; et
former une endoprothèse vasculaire tubulaire (57) à partir de la feuille étirée, comprenant l'endoprothèse vasculaire un mécanisme à glisser et verrouiller (slide-and-lock) permettant le pas de l'endoprothèse vasculaire d'un diamètre écrasé à un diamètre étiré et empêchant le recul radial à partir du diamètre étiré.

2. Procédé de la revendication 1, dans lequel la feuille de polymère est fabriquée en un polyester biodégradable choisi dans le group constitué de PLLA et PLGA.

3. Procédé de la revendication 1, dans lequel la feuille de polymère comprend ou est faite d'un polycarbonate dérivé de la tyrosine.

4. Procédé de la revendication 1, dans lequel le mécanisme à glisser et verrouiller comprend des modules (12', 12") circonférentiellement adjacents, comprenant les modules des éléments (14', 16'; 14", 16") radiaux à glisser et verrouiller longitudinalement adjacents qui permettent le glissement à sens unique des éléments radiaux du diamètre écrasé au diamètre étiré et empêchant le recul radial à partir du diamètre étiré.

5. Procédé de la revendication 1, comprenant en outre l'étirement de la feuille le long d'un deuxième axe d'étirement pour fournir une orientation biaxiale à la feuille, dans lequel l'axe cylindrique du tube est parallèle, perpendiculaire, ou forme un angle entre parallèle et perpendiculaire avec le deuxième axe d'étirement.

6. Procédé de la revendication 5, dans lequel l'axe d'étirement est perpendiculaire à l'axe cylindrique et le deuxième axe d'étirement est parallèle à l'axe cylindrique.

7. Procédé de la revendication 1, dans lequel la feuille est composée essentiellement d'un polymère bioabsorbable.

8. Procédé de la revendication 1, dans lequel le mécanisme à glisser et verrouiller est formé par découpe au laser de la feuille étirée.

9. Procédé de la revendication 1, dans lequel l'étirement est effectué à une température supérieure ou égale à la température de transition vitreuse (Tg) du polymère et inférieure à une température de fusion (Tm) du polymère.

10. Procédé de la revendication 1, dans lequel le polymère est semi-cristallin ou amorphe avec une température de transition vitreuse (Tg) au moins 10°C supérieure à la température du corps humain (Tbody)

11. Procédé de la revendication 10, comprenant en outre l'échauffement de la feuille de polymère de façon uniforme ou essentiellement uniforme à une température au moins 5 °C supérieure à la température de transition vitreuse (Tg) du polymère et tremper la feuille étirée à une température inférieure à la température de transition vitreuse (Tg) après l'étirement, dans lequel la réduction de la température empêche ou prévient la relaxation de l'orientation de la chaîne de polymère entraînée par l'étirement.

12. Procédé de la revendication 1, dans lequel la feuille de polymère est réticulée avant de l'étirement, dans lequel la réticulation empêche ou prévient la relaxation de l'orientation de la chaîne de polymère entraînée par l'étirement, étant le degré de réticulation inférieur à 5%.

13. Procédé de la revendication 1, comprenant en outre entraîner la réticulation de la feuille de polymère pendant ou après l'étirement, dans lequel la réticulation empêche ou prévient la relaxation de l'orientation de la chaîne de polymère entraînée para l'étirement.

14. Procédé de la revendication 12, dans lequel le polymère est semi-cristallin ou amorphe et a une température de transition vitreuse (Tg) supérieure à la température du corps humain (Tbody).

15. Procédé de la revendication 1, dans lequel la feuille de polymère comprend des nanoparticules qui sont dispersées dans le polymère.
